Europäisches Patentamt

European Patent Office

Office européen des brevets

(11) Publication number: **0 432 700 A2**

# EUROPEAN PATENT APPLICATION

(21) Application number: **90123713.1**

(22) Date of filing: **15.08.88**

(51) Int. Cl.⁵ **A61K 33/14**, A61K 33/04,
A61K 33/00, A61K 31/19,
A61K 31/60

This application was filed on 10 - 12 - 1990 as a divisional application to the application mentioned under INID code 60.

The title of the invention has been amended (Guidelines for Examination in the EPO, A-III, 7.3).

(30) Priority: **25.08.87 GB 8719988**

(43) Date of publication of application:
**19.06.91 Bulletin 91/25**

(60) Publication number of the earlier application in accordance with Art.76 EPC: **0 305 097**

(84) Designated Contracting States:
**AT BE CH DE ES FR GB GR IT LI LU NL SE**

(71) Applicant: **EFAMOL HOLDINGS PLC**
**Efamol House Woodbridge Meadows**
**Guildford Surrey GU1 1BA(GB)**

(72) Inventor: **Horrobin, David Frederick**
**2e Cedar Lodge, Lythe Hill Park**
**Haslemere, Surrey(GB)**

(74) Representative: **Cockbain, Julian et al**
**Frank B. Dehn & Co. Imperial House 15-19,**
**Kingsway**
**London WC2B 6UZ(GB)**

(54) Use of lithium compounds for the treatment of combination skin.

(57) There is provided the use of a physiologically tolerable lithium compound for the manufacture of an agent for use in a method of treatment of combination skin.

# NUTRITIONAL SUPPLEMENT

The present invention relates to the use of physiologically tolerable lithium compounds and in particular to the use of such compounds for the manufacture of nutritional supplements for use in combatting conditions associated with essential chemical deficiency.

Lithium compounds have been widely used in the treatment of manic depressive psychosis but have also been reported as being suitable for the treatment of other ailments. Thus Sherwin in US-A-3639625 suggests the use of topical lithium succinate compositions for the treatment of various skin diseases, Horrobin and Lieb in US-A-4386072 suggest the use of lithium compounds for the treatment of certain disorders of inflammation and immunity, EP-B-85579 (Efamol Ltd.) suggests the use of lithium compounds for the treatment of skin lesions, and EP-A-132126 (Efamol Ltd.) suggests the use of topical lithium-containing compositions in the treatment of seborrheic dermatitis, a fungal disease arising from excessive multiplication of a fungus which is normally present on the skin surface at tolerable levels.

Lithium treatment however has often been accompanied by a number of side-effects which are generally related to dosage levels and to the degree of accumulation. Particular side-effects which have occurred, especially in the treatment of manic depressive illness, include transient nausea, fine tremor, fatigue, muscular weakness, polydipsia and polyuria. Thus it is stated in Martindale The Extra Pharmacopoeia 28th Edition (1982) 1538 that the margin between the therapeutic and the toxic concentration of lithium is narrow, and that therefore it should be given under close medical supervision.

We have now surprisingly found that significantly depressed lithium plasma levels, relative to those of healthy subjects, are associated with a number of ailments and diseases, in other words that lithium appears to be an essential trace element.

Thus in healthy subjects lithium plasma levels may generally be in excess of 0.04 mM/l, whereas in subjects suffering from atopic eczema or seborrheic dermatitis mean plasma levels of below 0.025 mM/l are found. Alcoholics, patients with psoriasis, candidiasis, pityriasis and other fungal skin infections and sufferers from combination skin similarly exhibit depressed lithium plasma levels. (Combination skin is a troublesome and unsightly complaint manifested by excessive greasiness in certain skin areas, such as the forehead and the nose, and excessive dryness in other skin areas, such as the sides of the face).

We now propose therefore that conditions associated with essential chemical deficiency due to low lithium plasma levels may be countered or prevented from arising by the administration of a nutritional supplement containing a physiologically tolerable lithium compound.

Viewed from one aspect, the present invention thus provides the use of a physiologically tolerable lithium compound for the manufacture of a nutritional supplement for use in combatting conditions associated with essential mineral deficiency, more particularly lithium deficiency.

The nutritional supplement, which conveniently may be sterile, may be in a form adapted for enteral, parenteral or topical administration, but most preferably will be in a form adapted for oral ingestion or parenteral, e.g. iv, injection or infusion. Liquid preparations made with sterile or deionized water are particularly preferred. However, in another preferred embodiment the nutritional supplement may take the form of dietary supplement, such as a foodstuff. The nutritional supplement may alterna- tively be in a conventional pharmaceutical dosage form adapted for administration to the gastrointestinal tract. In this regard, forms such as tablets, coated tablets, capsules, powders, drops, suspensions, solutions, syrups and suppositories deserve particular mention. Nevertheless as lithium nutritional supplemen- tation may be achieved by parenteral or topical administration, for example by injection or by topical application (e.g. of an ointment, lotion, cream, paste or gel or the like), or by transdermal iontophoretic delivery, the nutritional supplement may be in the form of a composition adapted for one of these administration modes.

Where the nutritional supplement is prepared in a conventional pharmaceutical dosage form it may of course also contain conventional pharmaceutical carriers or excipients.

For parenteral administration by injection or infusion the nutritional supplements of the invention are preferably formulated as sterile solutions, emulsions or suspensions, e.g. in water for injections or in solutions in a lipid or lipid solvent, e.g. the alcohol analogues of the polyunsaturated fatty acids, discussed below.

For oral administration, the nutritional supplement may conveniently take the form of a foodstuff, for example a food or drink mix, into which a lithium compound is incorporated. The lithium containing nutritional supplement of the invention may particularly suitably be in the form of a so-called "complete" foodstuff, analogous to those which are prepared to serve as the major or sole source of nutrition for example for people wishing to loose weight, for post-operative patients, for elderly patients, for convalescents, or for individuals with specific dietary needs (e.g. patients with diabetes, coeliac disease or cystic fibrosis). For most people however a lithium supplemented foodstuff will preferably be of a type that is ingested daily in similar quantities, and for this reason the supplement will particularly conveniently comprise the lithium compound and a cereal or legume foodstuffs base. In an especially preferred embodiment, the nutritional supplement may be in the form of a breakfast cereal. In a preferred alternative, however, the nutritional supplement may take the form of a lithium-containing multi-vitamin multi- mineral preparation, for example in the form of tablets, capsules, or drops. In this regard, composi- tions are especially preferred which contain the lithium compound together with sources of one, some or all of the vitamins and the other essential elements, for example selected from vitamins A, $B_1$, $B_2$, $B_3$, $B_6$, $B_{12}$, C, D and E and calcium, copper, zinc, manganese and iron. In another embodiment, the lithium compound may be incorporated into an enteral or parenteral alimentation solution. The nutritional supplement of the invention may however contain the lithium compound as the sole active ingredient.

For topical administration, the nutritional supplement will again preferably be in a form adapted for regular application in substantially similar quantities and thus the lithium compound may particularly conveniently be incorporated within cosmetics, such as facial creams and ointments and the like.

The lithium compound used according to the invention may be any physiologically tolerable lithium compound that permits lithium uptake by the body. In general therefore, the preferred compounds will be lithium salts and in particular those salts which are at least sparingly water- soluble. Particularly suitable lithium compounds thus include lithium carbonate, lithium citrate, lithium chloride, lithium succinate, lithium sulphate, lithium salicylate, lithium acetylsalicylate, lithium orotate and the lithium salts of polyunsaturated fatty acids, especially the $C_{18-22}$ fatty acids and more especially the essential fatty acids. Thus it is especially preferred that the nutritional supplement contain at least one lithium salt of an essential fatty acid in the n-6 series (e.g. 18:2 n-6 (linoleic acid), 18:3 n-6 (gammalinolenic acid), 20:3 n-6 (dihomogammalinolenic acid), 20:4 n-6 (arachidonic acid), 22:4 n-6 (adrenic acid) and 22:5 n-6) or in the n-3 series (e.g. 18:3 n-3 (alpha linolenic acid), 18:4 n-3, 20:4 n-3, 20:5 n-3 (eicosa pentaenoic acid). 22:5 n-3 and 22:6 n-3 (docosahexa enoic acid)).

Most especially preferably the nutritional supplement will contain at least one essential fatty acid of the n-3 series, or a salt, most preferably the lithium salt thereof, and at least one essential fatty acid of the n-6 series, or a salt, again most preferably the lithium salt, thereof.

The essential fatty acids are essential nutrients which must be provided in the diet because they cannot be manufactured by the body. The main dietary essential fatty acids are linoleic and alphalinolenic acids but to be fully utilised the essential fatty acids must be metabolised along the n-3 or n-6 pathways, of which alphalinolenic and linoleic acids are the respective starting points.

In patients who are receiving no food or only insufficient food by the normal oral route the essential fatty acids must be provided by enteral or parenteral feeding.

On enteral adminstration, however the fatty materials may not be fully absorbed by patients having fat malabsorbtion. Such malabsorbtion is particularly likely to occur with patients with defective pancreatic or hepatic functions, with certain intestinal diseases or with cystic fibrosis. Essential fatty acids thus may be administered intravenously as parenteral nutrition supplements but at present this is generally done by administration of liquid emulsions, usually containing linoleic acid with or without alphalinolenic acid. Such liquid emulsions are not readily compatible with conventional aqueous fluids for parenteral nutrition. administration of liquid emulsions is not clear of side effects and there is particular concern regarding possible effects on the lungs, especially of infants.

We have found that the lithium salts of the essential fatty acids surprisingly are crystalline solids at ambient temperature which are highly soluble in water and alcohol. These properties facilitate preparation of compositions containing lithium or the essential fatty acids and thus the salts can be used to promote lithium and/or essential fatty acid uptake following administration. Where essential fatty acids are to be incorporated into the nutritional supplements of the invention they may accordingly be particularly conveniently incor- porated as the lithium salts. However the essential fatty acids may also conveniently be incorporated in the form of salts with other counterions, especially the sodium and potassium salts which are also water soluble. Particularly conveniently, the nutritional supplement may contain two or more essential fatty acid sodium, lithium or potassium salts.

Where the nutritional supplements are for parenteral administration and contain essential fatty acid salts they are preferably formulated as aqueous solutions, and preferably are stored under an inert atmosphere, e.g. in nitrogen flushed vials, buffered to a pH in the range 8.5 to 9.0, e.g. with a physiologically acceptable acid such as citric acid. Moreover, such solutions preferably contain an antioxidant, e.g. a fatty acid derivative of ascorbic acid such as ascorbyl palmitate, a tocopherol (e.g. gamma tocopherol or a mixed tocopherol) or butylated hydroxy toluene.

The essential fatty acid supply required to prevent essential fatty acid deficiency is probably within the range 0.5-8% of total daily calorie intake, especially 1-3% (or 1-5% during periods of metabolic stress such as rapid growth, pregnancy, lactation or injury). In the case of orally or parenterally administrable nutritional supplements, the nutritional supplements are preferably administered at a daily or one-off dosage of the essential fatty acid or salt thereof of 1 to 100,000 mg, especially 1-50000 mg, preferably 100-10000 mg, conveniently in dosage units of 50, 100, 250, 500 or 1000 mg. For topical administration, concentrations of the essential fatty acids or salts thereof may conveniently be 0.001 to 50%, for example from 0.01 to 30%, preferably 0.1 to 5%, be weight.

Where the nutritional supplement of the invention contains a lithium salt of a polyunsaturated fatty acid and is in a form adapted for oral administration, the lithium salt in the supplement is preferably provided with a gastric juice resistant release delaying coating, e.g. a Eudragit coating as supplied by Röhm GmbH of Darmstadt.

The lithium content in the nutritional supplement will be selected according to the nature of the supplement and its administration route but in general will be within the range 1 ppb to 30% by weight of lithium, preferably 1 ppm to 20% lithium, especially preferably 0.001 to 10% lithium and particularly preferably up to 1% lithium. Obviously, where the nutritional supplement is in the form of a complete foodstuff, the lithium content will be towards the lower ends of the ranges specified above, for example it may conveniently be in the range 1 ppb to 10 ppm, preferably 10 ppb to 1 ppm. Thus, for example in complete foods which might be administered in a dose of 500g/day, a lithium content of 5mg might be contemplated. More particularly, the daily dosage will generally be such that the adult body receives from 1 microgram to 50 mg, preferably 1 to 10 mg, of lithium per day, and it will preferably be such as to maintain the lithium plasma level above 0.04 mM/l.

Viewed from a further aspect, the invention thus provides a nutritional supplement comprising a foodstuffs base with included therein a physiologi cally tolerable lithium compound in a concentration such that said supplement contains from 1 ppb to 1% by weight of lithium. Nutritional supplements prepared from sterilized or deionized base materials would be particularly preferred.

Viewed from a yet further aspect, the invention also provides a nutritional supplement comprising a sterile composition containing a physiologically tolerable lithium compound together with four or more, preferably eight or more, vitamins or minerals, for example essential vitamins or minerals selected from the group consisting of vitamins A, $B_1$, $B_2$, $B_3$, $B_6$, $B_{12}$, C, D and E and physiologically tolerable calcium, copper, zinc, manganese and iron compounds, and optionally together with at least one physiologi- cally acceptable carrier or excipient.

Viewed from a still further aspect, the invention also provides a nutritional supplement comprising a parenteral or enteral alimentation solution containing a physiologically tolerable lithium compound.

The nutritional supplement may be used to combat a wide range of conditions associated with essential chemical deficiency, in particular conditions which appear to be associated with immune system malfunction and especially conditions such as combina- tion skin, atopic eczema, psoriasis, seborrheic dermatitis, candidiasis, pityriasis, skin fungal infections and conditions associated with alcoholism.

Thus viewed from another aspect, the invention provides a method of treatment of the human or animal body to combat conditions associated with essential mineral deficiency, said method comprising administering to the body an effective amount of a nutritional supplement comprising a physiologically tolerable lithium compound.

Viewed from a further aspect the invention also provides a method of treatment of a subject suffering from alcoholism, said method comprising administering to said subject a physiologically tolerable lithium compound.

Viewed from a still further aspect, the invention provides a method of treatment of a subject having combination skin, said method comprising administering to said subject, preferably by the topical application of a cosmetic composition, a physiologically tolerable lithium compound.

The present invention will now be illustrated further by the following non-limiting Examples:

Example 1
Foodstuffs Composition

A breakfast cereal, for use as a nutritional supplement, contains the following ingredients in each 30 g serving:

| | |
|---|---|
| Vitamin A | 4000 iU |
| Vitamin B$_1$ | 1 mg |
| Vitamin B$_2$ | 1mg |
| Vitamin C | 50mg |
| Vitamin D | 400 iU |
| Calcium carbonate | 5mg |
| Lithium carbonate | 20mg |
| Rolled oats    ad | 30 g |

Example 2

Multi-Vitamin/Mineral Tablet

Multi-vitamin/mineral tablets for daily ingestion each contain the following ingredients:

| | |
|---|---|
| Vitamin A | 4000 iU |
| Vitamin B$_1$ | 1.5 mg |
| Vitamin B$_2$ | 1 mg |
| Vitamin B$_6$ | 1 mg |
| Vitamin B$_{12}$ | 2 microgram |
| Vitamin C | 40 mg |
| Vitamin D | 400 iU |
| Vitamin E | 4 mg |
| Calcium carbonate | 5 mg |
| Lithium citrate | 50 mg |
| Iron (II) carbonate | 10 mg |
| Manganese sulphate | 1 mg |
| Nicotinamide | 15 mg |
| Tableting base    ad | 450 mg |

The tablet components are mixed and compressed to form biconvex tablets which are then coated in a conventional manner. If desired lithium gamma- linolenate precoated with a gastric juice resistant release delaying coating (e.g. a Eudragit coating) or incorporated into a matrix of such a coating material and ground to a powder may be incorporated into the multi-vitamin/mineral tablet in place of the lithium carbonate.

Example 3

Cosmetic Composition

A night cleansing composition is formed by mixing three parts by weight of lithium succinate (or lithium gammalinolenate) with ninety seven parts by weight of a cosmetic night cleansing cream base.

Example 4
Body Lotion

A body lotion is formed by mixing 4 parts by weight of lithium gammalinolenate with 96 parts by weight of a body lotion base.

Example 5
Face Cream

A day face cream is formed by mixing 3 parts by weight of lithium citrate with 97 parts by weight of a face cream base.

Example 6
Complete Foodstuff

A complete foodstuff, particularly suited for adminis tration to geriatric or convalescent subjects at a daily dosage of about 500 g/day, is prepared by admixing 50mg of lithium carbonate (or 70 mg lithium gamma linolenate) together with 500g of a complete foodstuffs composition containing all other major essential nutrients.

Example 7
Parenteral Alimentation Solution

A solution for lithium-supplemented parenteral alimentation is prepared by dissolving 20mg of lithium citrate (or 1 g lithium linoleate or 1 g lithium linoleate and 0.3 g lithium alpha linolenate or 1 g lithium linoleate, 0.3 g lithium alpha linolenate, 0.3 g lithium gamma linolenate and 0.3 g lithium eicosapentaenoate) in 500ml of a parenteral alimentation solution (e.g. Vamin N or Vamin Glucose, optionally admixed about 13:1 by volume with Ped-El, (Vamin and Ped-El are products available from KabiVitrum Ltd, Uxbridge, United Kingdom).

Example 8
Tablets

Tablets are prepared from lithium citrate, lithium carbonate and lithium succinate and conventional tabletting aids. Each tablet contains respectively 10, 20, 5, 10 and 50mg of lithium citrate, lithium citrate, lithium carbonate, lithium carbonate or lithium succinate.

Example 9
Complete liquid food

A complete liquid food, for adminstration by enteral tube for example, is prepared for use by geriatrics or convalescents by admixing 50 mg of lithium citrate (or 25 mg of lithium carbonate or 150 mg of lithium gamma linolenate) into 500 g of a conventional complete foodstuffs composition containing all other major essential nutrients.

Example 10
Injection solution

Sterile ampoules for injection are prepared containing 30 mg lithium citrate or 100 mg lithium linoleate dissolved in water for injections buffered to pH 8.5 to 9.0 with citric acid. The contents may be injected directly or, preferably, may be added to fluids for intravenous administration.

Example 11
Multi-vitamin/mineral tablets

Multi-vitamin and mineral tablets for daily ingestion each contain the following ingredients:

6

| | |
|---|---|
| Vitamin A | 750 microgram |
| Vitamin $B_1$ | 1.2 mg |
| Vitamin $B_2$ | 1.6 mg |
| Vitamin $B_6$ | 1 mg |
| Vitamin $B_3$ | 2 mg |
| Vitamin $B_{12}$ | 2 microgram |
| Vitamin C | 25 mg |
| Vitamin D | 2.5 microgram |
| Vitamin E | 4.5 mg |
| Iron (II) carbonate | 11 mg |
| Calcium carbonate | 4 mg |
| Lithium carbonate | 10 mg |
| Manganese sulphate | 1 mg |
| Nicotinamide | 15 mg |
| Tabletting base | 470 mg |

The tablets are prepared analogously to those of Example 2.

Example 12
Soup mix

A conventional dried soup mix (single serving) is adjusted by the addition of 5 g lithium carbonate (or 40 g of lithium linoleate, gammalinolenate or eicosapentaenoate)

Example 13
Baby food

A baby food (e.g. of the type pre-prepared and packaged in bottles or tins in individual servings) is adjusted by the addition of 2 mg of lithium carbonate (or 4 mg of lithium citrate or 10 mg of lithium gammalinolenate) per 200 kcal calorific value. For a canned vegetable meal, having a calorific value of 89 kcal for a service of 128 g, 0.89 mg of lithium carbonate may thus be uniformly dispersed within the can's contents.

Example 14
Orally administrable capsules

Appropriately sized hard or soft gelatin capsules are each filled with 250, 500 or 1000 mg of lithium gamma-linolenate and optionally are provided with an enteric coating.
Alternatively, lithium eicosapentaenoate, lithium arachidonate, or lithium dihomo-gamma-linolenate may be used in place of the lithium gamma-linolenate or a combination of two or more of these four lithium salts may be used.

Example 15
Orally administrable capsules

Appropriately sized hard or soft gelatin capsules are each filled with 100 mg of lithium eicosapentaenoate and 400 mg of gamma-linolenyl alcohol and optionally are provided with an enteric coating.

Example 16
Orally administrable capsules

7

Appropriately sized hard or soft gelatin capsules are each filled with 50 mg of lithium arachidonate and 250 mg of dihomo-gamma-linolenyl alcohol and optionally are provided with an enteric coating.

Example 17
Orally administrable capsules

Appropriately sized hard or soft gelatin capsules are each filled with 50 mg of lithium eicosapentaenoate, 50 mg of lithium arachidonate and 400 mg of gamma-linolenyl alcohol and optionally are provided with an enteric coating.

Example 18
Orally administrable capsules

Appropriately sized hard or soft gelatin capsules are each filled with 50 mg of lithium gamma-linolenate, 50 mg of eicosapentaenyl alcohol and 100 mg of dihomo-gamma linolenic acid and optionally are provided with an enteric coating.

Example 19
Orally administrable capsules

Appropriately sized hard or soft gelatin capsules are each filled with 50 mg of lithium arachidonate, 50 mg of docosahexaenyl alcohol (22:6n-3) and 220 mg of evening primrose oil-derived triglycerides and optionally provided with an enteric coating.

Example 20
Parenterally administrable solutions

A solution is prepared by dissolving 5g of lithium gamma-linolenate, alone or with 5g of lithium arachido nate, lithium eicosapentaenoate, or lithium docosahexa enoate, in 500ml of saline or glucose solution for intravenous administration. The essential fatty acid lithium salt(s) may be conveniently prepared in glass vials each containing 5g of the salt which can then be dissolved in sterile water and added to the intravenous solution. Compositions containing the same lithium salt(s) can be prepared for intramuscular or subcutaneous injection.

Example 21
Solutions for enteral or rectal administration

A solution is prepared by dissolving 5g of lithium gamma-linolenate or a combination of essential fatty acid lithium in 100ml of an enteral or rectal administration solution.

Example 22
Parenterally administrable emulsions

An emulsion is prepared by emulsifying one volume of an oil phase comprising 5 g of lithium eicosapenta- enoate and 5 g of lithium gamma-linolenate dissolved in 100 g of corn oil with ten volumes of an aqueous phase comprising a 1% lecithin and 2% glycerol aqueous solution.

Example 23
Parenterally administrable emulsions

An emulsion is prepared by emulsifying one volume of an oil phase comprising 10 g of lithium dihomo gamma-linolenate and 5 g of eicosapentaenyl alcohol dissolved in 100 g of corn oil with seven volumes of an aqueous phase comprising a 1.5% lecithin and 2% glycerol aqueous solution.

The eicosapentaenyl alcohol may if desired be replaced by gamma-linolenyl alcohol or any of the other alcohol analogues of the essential fatty acids. Similarly other essential fatty acid lithium salts may be used in place of lithium dihomo-gamma-linolenate.

Example 24
Orally administrable tablets

Tablets are prepared in a conventional manner from lithium gamma-linolenate, arachidonate, eicosapenta enoate or any other essential fatty acid lithium salt admixed with a physiologically acceptable tabletting aid. The mixture is compressed to yield tablets each containing 500 mg of the lithium salt. These tablets may then if desired be provided with a gastric juice resistant enteric coating.

Example 25
Cosmetic composition

Cosmetic compositions, e.g. creams, lotions or the like, may be prepared by mixing into a conventional cosmetic composition sufficient lithium gamma-linolenate and lithium eicosapentaenoate to produce a composition containing containing 3% lithium gamma-linolenate and 1% lithium eicosapentaenoate. Where the composition contains no aqueous, alcoholic or lipid solvents, the lithium salts are preferably used in finely divided form.

Example 26
Skin and hair care compositions

Skin and hair care compositions, such as lotions, creams or shampoos, may be prepared by mixing into a conventional skin or hair care composition sufficient lithium gamma-linolenate and lithium eicosapentaenoate to yield a composition containing 5% lithium gamma-linolenate and 3% lithium eicosapentaenoate.

Example 27
Injection solutions for addition to parenteral nutrition fluids

Solutions are prepared containing:

| | | |
|---|---|---|
| (a) | lithium linoleate | 1g |
| | ethanol/0.9% saline (50/50 by volume) | 10ml |
| (b) | lithium linoleate | 1g |
| | lithium alpha-linolenate | 0.3g |
| | ethanol/0.9% saline (50/50 by volume) | 10ml |
| (c) | lithium linoleate | 1g |
| | lithium alpha-linolenate | 0.3g |
| | lithium gamma-linolenate | 0.3g |
| | lithium eicosapentaenoate | 0.3g |
| | ethanol/0.9% saline (50/50 by volume) | 10ml |

These solutions are each filled into vials under aseptic conditions and sealed therein. The contents of such vials may be added to 100ml or 500ml or other sizes of bottles of aqueous fluids for parenteral nutrition which do not already contain essential fatty acids.

In this Example, in place of the essential fatty acid lithium salts specified other lithium salts may instead be used, for example salts of n-6 essential fatty acids such as lithium dihomogamma-linolenate, lithium arachidonate, lithium adrenate and lithium docosapenta enoate(22:5n-6) and salts of the n-3 essential fatty acids such as lithium stearidonate, lithium 20:4n-3, lithium docosapentaenoate (22:5n-3) and lithium docosahexaenoate (22:6n-3).

Example 28

9

Parenteral solutions

5g of lithium gamma-linolenate or lithium eicosapenta enoate (or another essential fatty acid lithium salt) is dissolved in 500 ml of physiological saline containing 5% glucose and the solution is filled into bottles, flasks or bags under aseptic conditions. Such containers may then be used for parenteral administration.

Examples 29 to 35
Nutritional solution supplements

Nutritional solution supplements, for addition to paren teral or enteral nutrition solutions are prepared by dissolving the following materials in water for injec tions, optionally containing ethanol as up to 50% of the solvent. The solutions are optionally buffered to pH 8.5 to 9.0 and optionally further contain an antioxidant. The supplement solutions are sterile filled into dark glass ampoules which are flushed with and sealed under nitrogen.

| Example No. | 29 | 30 | 31 | 32 | 33 | 34 | 35 |
|---|---|---|---|---|---|---|---|
| Sodium linoleate | | 200 | 250 | 150 | 300 | 500 | 300 |
| Sodium gammalinolenate | | | 100 | 200 | | | 100 |
| Sodium dihomogamma-linolenate | | | | | 100 | | |
| Sodium arachidonate | | | 100 | | 100 | | |
| Sodium alphalinolenate | | 250 | 100 | | | | |
| Potassium linoleate | | 200 | | 200 | 200 | | 300 |
| Potassium alphalinolenate | | | | | 100 | 250 | 100 |
| Potassium eicosapentaenoate | 100 | | | | | 100 | 50 |
| Potassium docosahexaenoate | 100 | | | | | 100 | 50 |
| Lithium linoleate | 500 | 200 | 250 | 200 | 200 | | |
| Lithium gammalinolenate | 100 | 250 | | | 100 | 150 | |
| Lithium dihomogamma-linolenate | 100 | | 100 | | | 50 | 100 |
| Lithium arachidonate | 100 | | | | | 50 | 50 |
| Lithium alphalinolenate | 250 | 100 | 150 | | | | |
| Lithium eicosapentaenoate | 100 | | 100 | 100 | 100 | | 50 |
| Lithium docosahexaenoate | 100 | | 100 | 100 | 100 | | 50 |

(The figures are in mg).

**Claims**

1. The use of a physiologically tolerable lithium compound for the manufacture of an agent for use in a method of treatment of combination skin.

2. Use as claimed in claim 1 of a lithium salt selected from lithium carbonate, lithium citrate, lithium chloride, lithium succinate, lithium sulphate, lithium salicylate, lithium acetylsalicylate and lithium orotate.

3. Use as claimed in claim 1 or claim 2 for the manufacture of an agent adapted for topical administration.

4. Use as claimed in claim 3 for the manufacture of an agent in the form of a cosmetic composition.

5. Use as claimed in claim 1 or claim 2 for the manufacture of an agent in the form of an orally administrable alimentation solution.

6. Use as claimed in Claim 1 or Claim 2 for the manufacture of an agent in the form of a sterile composition containing a physiologically tolerable lithium compound together with four or more vitamins or minerals, and optionally together with at least one physiologically acceptable carrier or excipient.

7. Use as claimed in claim 6 wherein said agent comprises four or more essential vitamins or minerals selected from the group consisting of vitamins A, $B_1$, $B_2$, $B_3$, $B_6$, $B_{12}$, C, D and E and physiologically tolerable calcium, copper, zinc, manganese and iron compounds.

8. Use as claimed in any one of claims 1 to 7 for the manufacture of an agent comprising 1 ppb to 10% by weight lithium.

9. Use as claimed in Claim 1 or Claim 2 for the manufacture of an agent in the form of a complete foodstuff containing from 1 ppb to 1% by weight of lithium.

10. Use as claimed in any one of claims 1 to 9 for the manufacture of an agent additionally comprising one or more essential fatty acids or physiologically tolerable salts thereof.